(19) Europäisches Patentamt
European Patent Office
Office européen des brevets

(11) Veröffentlichungsnummer: **0 025 548**
**B1**

(12) **EUROPÄISCHE PATENTSCHRIFT**

(45) Veröffentlichungstag der Patentschrift: **16.06.82**

(51) Int. Cl.³: **C 07 C 127/15**

(21) Anmeldenummer: **80105226.7**

(22) Anmeldetag: **03.09.80**

(54) **Verfahren zur Herstellung von Alkylharnstoffen.**

(30) Priorität: **14.09.79 DE 2937331**

(43) Veröffentlichungstag der Anmeldung: **25.03.81 Patentblatt 81/12**

(45) Bekanntmachung des Hinweises auf die Patenterteilung: **16.06.82 Patentblatt 82/24**

(84) Benannte Vertragsstaaten: **BE DE FR GB IT NL**

(56) Entgegenhaltungen:
**US-A-2 253 528**

**CHEMICAL ABSTRACTS, volume 80, Nr. 21, 27. Mai 1974 Zusammenfassung 120351p, Seite 387**

(73) Patentinhaber: **BAYER AG, Zentralbereich Patente, Marken und Lizenzen, D-5090 Leverkusen 1, Bayerwerk (DE)**

(72) Erfinder: **Findeisen, Kurt, Dr., In der Follmühle 10, D-5068 Odenthal 2 (DE)**
Erfinder: **Freimuth, Reinhard, Dr., Pütrichstrasse 3, D-8000 München 80 (DE)**
Erfinder: **Wagner, Kuno, Dr., Am Kiesberg 8, D-5090 Leverkusen (DE)**

**EP 0 025 548 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

# Verfahren zur Herstellung von Alkylharnstoffen

Die vorliegende Erfindung betrifft ein neues Verfahren zur Herstellung von Alkylharnstoffen durch direkte Umsetzung von Alkylaminen mit Harnstoff.

N-Alkylharnstoffe und N,N-Dialkylharnstoffe, insbesondere N,N-Dimethyl- und N,N-Diethylharnstoff sind bekannt. Diese Verbindungen stellen insbesondere interessante Vor- und Zwischenprodukte zur Herstellung von Isocyanaten, Wirkstoffen, Polyurethanen und Raketentreibstoff (als N,N-Dimethylhydrazin, J. Russ. Phys. Chem. Soc. *37*, 1) dar.

Es wurden Synthesewege wie Umsetzung des Carbamidsäurechlorides mit Ammoniak, Salze des Amins mit Alkalimetall-Cyanaten oder Amin mit Nitroharnstoff vorgeschlagen (Houben-Weyl, Georg Thieme Verlag, Stuttgart 1952, Band VIII, 153). Die hierzu verwendeten Ausgangsprodukte sind relativ kostspielig, physiologisch bedenklich und schwer zugänglich. Auch die Herstellung durch Reaktion von Alkyl- oder Dialkylamin mit Harnstoff wurde beschritten. In der US-Patentschrift 2 253 528 wird dazu entweder in einem geschlossenen Reaktionskessel unter Überdruck oder, wenn der Siedepunkt des Amins gleich oder höher als die für die Reaktion benötigte Temperatur liegt, in einem offenen System gearbeitet. Auch wurde vorgeschlagen, die Reaktion in einer aufwendigen Gegenstromapparatur möglichst unter Druck durchzuführen (DE-AS 1 111 613) oder gasförmiges Amin in geschmolzenen Harnstoff einzuleiten (DE-PS 869 640). Da Harnstoff im geschmolzenen Zustand bekanntlich leicht zu Nebenreaktionen neigt (z. B. Biuretbildung), ist das Verfahren der letztgenannten Vorveröffentlichung mit dem Nachteil behaftet, dass nur unreine Verfahrensprodukte in schlechter Ausbeute erhalten werden.

Gemäss DE-PS 855 551 oder US-PS 3 937 727 erfolgt die Umsetzung zwischen Harnstoff und Methyl- bzw. Dimethylamin unter Druck in wässrigem bzw. wasserfreiem Milieu. Diese bekannten unter äusserem Druck ablaufenden Verfahren des Standes der Technik sind mit dem Nachteil behaftet, dass Autoklaven bzw. aufwendige technische Apparaturen verwendet werden müssen.

Der vorliegenden Erfindung lag daher die Aufgabe zugrunde, ein neues Verfahren zur Verfügung zu stellen, welches nicht mit den genannten Nachteilen der bekannten Verfahren behaftet ist, d. h. insbesondere, welches unabhängig vom Siedepunkt des eingesetzten Amins unter atmosphärischem Druck die Herstellung von unsymmetrischen Alkylharnstoffen in hoher Reinheit und Ausbeute gestattet, so dass die grosstechnische Herstellung dieser Verbindungen stark vereinfacht werden kann.

Diese Aufgabe konnte überraschenderweise dadurch gelöst werden, dass bei der Herstellung der unsymmetrischen Alkylharnstoffe durch Umsetzung von Harnstoff mit aliphatischen Mono- oder Diaminen bestimmte Verdünnungsmittel mitverwendet werden. Die erfindungsgemäss aufgefundene Lösung der gestellten Aufgabe kann als überraschend bezeichnet werden, da nicht zu erwarten war, dass die Mitverwendung derartiger Verdünnungsmittel die Tendenz von Harnstoff zur Bildung von unerwünschten Nebenprodukten beeinträchtigt.

Gegenstand der vorliegenden Erfindung ist ein Verfahren zur Herstellung von Alkyl-substituierten Harnstoffen der Formel

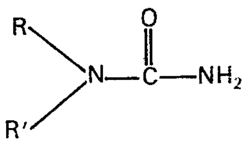

in welcher

R und R' für gleiche oder verschiedene Reste stehen und Wasserstoff oder gesättigte aliphatische Kohlenwasserstoffreste bedeuten, wobei jedoch mindestens einer der Reste für einen gesättigten aliphatischen Kohlenwasserstoffrest steht,

durch drucklose Umsetzung bei 110 bis 170 °C von Harnstoff mit einem Amin mit einem unter Normaldruck mindestens 10 °C unterhalb der Reaktionstemperatur liegenden Siedepunkt der Formel

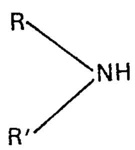

in welcher

R und R' die obengenannte Bedeutung haben,

wobei die Mengenverhältnisse der Reaktionspartner so gewählt werden, dass für jedes Mol Harnstoff mindestens 1 Mol Amin zur Umsetzung gelangt, dadurch gekennzeichnet, dass man die Umsetzung in Gegenwart von 20 bis 95 Gew.-%, bezogen auf die Gesamtmenge aller Reaktionspartner und Hilfsmittel, einer unter den Reaktionsbedingungen inerten, unter Normaldruck oberhalb der Reaktionstemperatur siedenden organischen Flüssigkeit durchführt, die für den Harnstoff und das Verfahrensprodukt ein Dispergier- oder Lösungsmittel und für das Amin ein Lösungsmittel darstellt.

Unter «druckloser Umsetzung» ist hierbei eine Umsetzung zu verstehen, bei der ohne Anwendung eines äusseren Drucks, d. h. ohne Überdruck gearbeitet wird.

Ausgangsverbindungen für das erfindungsgemässe Verfahren sind Amine der Formel

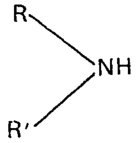

in welcher
R und R' die bereits genannte Bedeutung haben.

Vorzugsweise werden beim erfindungsgemässen Verfahren solche Amine der genannten Formel eingesetzt, für welche R und R' für Wasserstoff oder gesättigte aliphatische Kohlenwasserstoffreste mit 1 bis 5 Kohlenstoffatomen, insbesondere 1 oder 2 Kohlenstoffatome steht, wobei jeweils mindestens einer der Reste R oder R' für einen derartigen Kohlenwasserstoffrest steht. Die beim erfindungsgemässen Verfahren einzusetzenden Amine weisen im übrigen unter Normaldruck einen mindestens 10 °C unter der Reaktionstemperatur liegenden Siedepunkt auf.

Beispiele geeigneter Amine sind z. B. Methylamin, Ethylamin, n-Propylamin, n-Pentylamin, Dimethylamin, Diethylamin, Di-iso-butylamin oder Methyl-ethylamin. Dimethylamin und Diethylamin sind die bevorzugten beim erfindungsgemässen Verfahren einzusetzenden Amine.

Das erfindungsgemässe Verfahren wird in Gegenwart von Verdünnungsmitteln eingesetzt. Bei diesen Verdünnungsmitteln handelt es sich um organische Flüssigkeiten, die unter den Bedingungen des erfindungsgemässen Verfahrens inert sind, unter Normaldruck einen oberhalb der Reaktionstemperatur liegenden Siedepunkt aufweisen, und die für den Harnstoff bzw. das Verfahrensprodukt ein Lösungs- oder Dispergiermittel und für das Amin ein Lösungsmittel darstellen. Beispiele geeigneter Verdünnungsmittel sind Chlorbenzol, Dichlorbenzole, Trichlorbenzole, Toluol, Xylole wie o- oder p-Xylol bzw. technische Xylolgemische, Sulfolan, Benzonitril, Nitrobenzol, N-Methylpyrrolidon oder Diethylenglykolmonomethylether. Zu den besonders gut geeigneten Verdünnungsmitteln gehören Dichlorbenzole, N-Methylpyrrolidon oder Diethylenglykolmonomethylether.

Bei der Durchführung des erfindungsgemässen Verfahrens weden pro Mol Harnstoff mindestens 1 Mol, vorzugsweise 1 bis 2 Mol, Amin eingesetzt. Das Verdünnungsmittel wird in einer Menge von 20 bis 95, vorzugsweise 40 bis 85 Gew.-%, bezogen auf die Gesamtmenge aller Reaktionspartner und Hilfsmittel, eingesetzt.

Das erfindungsgemässe Verfahren wird bei 110 bis 170 °C, vorzugsweise 115 bis 150 °C, insbesondere 120 bis 135 °C, durchgeführt, wobei kein Druck zur Anwendung gelangt, so dass der entstehendes Ammoniak jederzeit frei entweichen kann.

Zur Durchführung des erfindungsgemässen Verfahrens wird das gegebenenfalls in Verdünnungsmittel gelöste Amin in eine auf die Reaktionstemperatur erhitzte Lösung oder Dispersion des Harnstoffs in Verdünnungsmittel eindosiert. Der Verlauf der Reaktion kann an der Menge des spontan entstehenden Ammoniaks erkannt werden. Die Verfahrensprodukte können nach beendigter Umsetzung aus dem Reaktionsgemisch durch Kristallisation oder nach destillativer Entfernung des Verdünnungsmittels als Rückstand gewonnen werden.

Das erfindungsgemässe Verfahren gestattet auf besonders einfache Weise die Herstellung von unsymmetrischen Alkyl- oder Dialkylharnstoffen, für welche bislang noch keine technisch völlig zufriedenstellende Herstellungsweise bekannt geworden ist.

In den nachfolgenden Beispielen beziehen sich alle Prozentangaben auf Gewichtsprozente.

Beispiel 1

1,2 kg (20 Mol) Harnstoff werden in 2,5 l trockenem o-Dichlorbenzol suspendiert. Man heizt zügig auf 135 °C Innentemperatur, wobei schon ein langsamer Strom Dimethylamin eingeleitet wird. Bei Erreichen der Reaktionstemperatur verstärkt man unter Kühlen die Zugabe des Dimethylamins und beendet sie, wenn 1,08 kg (24 Mol) eingeleitet sind. Das nach Abkühlen auf Raumtemperatur auskristallisierende Reaktionsprodukt wird abgesaugt und mit 1 l o-Dichlorbenzol und 2 l Alkohol gewaschen. Man erhält 1550 g (88% der Theorie) N,N-Dimethylharnstoff vom Fp.: 182—183 °C. Durch Isolierung des in der Mutterlauge gelösten Verfahrensprodukts nach deren Eindampfen kann die Ausbeute auf 97% erhöht werden.

Beispiel 2

180 g (3 Mol) Harnstoff werden in 500 ml trokkenem Xylol suspendiert. Man heizt zügig auf 130—135 °C Innentemperatur, wobei schon langsam eine Lösung von 219 g (3Mol) Diethylamin in 250 ml Xylol zudosiert wird. Beim Erreichen der Reaktionstemperatur verstärkt man unter Rühren die Zugabe der Diethylamin-Lösung. Nach Zugabe der Gesamtmenge der Aminlösung wird das Reaktionsgemisch auf die Hälfte seines Volumens eingeengt. Anschliessend lässt man auf Raumtemperatur abkühlen und saugt das auskristallisierende Reaktionsprodukt ab. Man erhält 290 g (83% der Theorie) N,N-Diethylharnstoff vom Fp.: 67 °C.

Beispiel 3

180 g (3 Mol) Harnstoff werden in 500 ml trokkenem Xylol suspendiert. Man heizt zügig auf 130—135 °C Innentemperatur, wobei schon langsam ein Gemisch aus 303 g (3 Mol) Dipropylamin und 250 ml Xylol zudosiert wird. Beim Erreichen der Reaktionstemperatur verstärkt man unter Rühren die Zugabe des Dipropylamin-Gemisches. Nach Zugabe der Gesamtmenge der Aminlösung wird das Reaktionsgemisch auf die Hälfte seines Volumens eingeengt. Anschliessend lässt man auf Raumtemperatur abkühlen und saugt das auskristallisierende Reaktionsprodukt ab. Man erhält 345 g (80% der Theorie) N,N-Dipropylharnstoff vom Fp.: 69 °C.

Beispiel 4

120 g (2 Mol) Harnstoff werden in 400 ml trokkenem Xylol suspendiert. Man heizt zügig auf

120–125 °C Innentemperatur, wobei schon ein langsamer Strom Methylamin eingeleitet wird. Beim Erreichen der Reaktionstemperatur verstärkt man unter Rühren die Zugabe des Methylamins und beendet sie, wenn 120 g (3,9 Mol) eingeleitet sind. Das Reaktionsprodukt wird aus der erkalteten Lösung abgetrennt und mit 100 ml Xylol gewaschen. Man erhält 117 g (79% der Theorie) Methylharnstoff vom Fp.: 96 °C.

Beispiel 5

120 g (2 Mol) Harnstoff werden in 500 ml trockenem Xylol suspendiert. Man heizt zügig auf 120–125 °C Innentemperatur, wobei schon ein langsamer Strom Ethylamin eingeleitet wird. Beim Erreichen der Reaktionstemperatur verstärkt man unter Rühren die Zugabe des Ethylamins und beendet sie, wenn 125 g (2,75 Mol) eingeleitet sind. Das Reaktionsprodukt wird, nachdem man im Vakuum etwas eingeengt hat, aus der erkalteten Lösung abgesaugt und mit wenig kaltem Xylol gewaschen. Man erhält 147 g (83% der Theorie) Ethylharnstoff vom Fp.: 86 °C.

Beispiel 6

120 g (2 Mol) Harnstoff werden im 350 ml trockenem Xylol suspendiert. Man heizt zügig auf 120–125 °C Innentemperatur, wobei schon langsam ein Gemisch aus 120 g (2,05 Mol) Propylamin und 200 ml Xylol zudosiert wird. Beim Erreichen der Reaktionstemperatur verstärkt man unter Rühren die Zugabe des Propylamin-Gemisches. Nach beendeter Aminzugabe wird das Reaktionsgemisch auf die Hälfte seines Volumens eingeengt. Anschliessend lässt man auf Raumtemperatur abkühlen, filtriert das auskristallisierende Reaktionsprodukt ab und wäscht es mit wenig kaltem Xylol. Man erhält 165 g (81% der Theorie) Propylharnstoff vom Fp.: 101 °C.

Beispiel 7

120 g (2 Mol) Harnstoff werden in 350 ml trockenem Xylol suspendiert. Man heizt zügig auf 120–125 °C Innentemperatur, wobei schon langsam ein Gemisch aus 175 g (2 Mol) n-Amylamin und 200 ml Xylol zudosiert wird. Beim Erreichen der Reaktionstemperatur verstärkt man unter Rühren die Zugabe des Amylamin-Gemisches. Nach beendeter Aminzugabe wird das Reaktionsgemisch auf die Hälfte seines Volumens eingeengt. Anschliessend lässt man auf Raumtemperatur abkühlen, filtriert das auskristallisierende Reaktionsprodukt ab und wäscht es mit wenig kaltem Xylol. Man erhält 229 g (88% der Theorie) n-Amylharnstoff vom Fp.: 94 °C.

**Patentansprüche**

1. Verfahren zur Herstellung von Alkyl-substituierten Harnstoffen der Formel

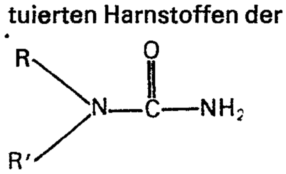

in welcher

R und R′ für gleiche oder verschiedene Reste stehen und Wasserstoff oder gesättigte aliphatische Kohlenwasserstoffreste bedeuten,

wobei jedoch mindestens einer der Reste für einen gesättigten aliphathischen Kohlenwasserstoffrest steht, durch drucklose Umsetzung bei 110 bis 170 °C von Harnstoff mit einem Amin mit einem unter Normaldruck mindestens 10 °C unterhalb der Reaktionstemperatur liegenden Siedepunkt der Formel

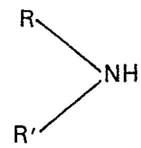

in welcher

R und R′ die obengenannte Bedeutung haben,

wobei die Mengenverhältnisse der Reaktionspartner so gewählt werden, dass für jedes Mol Harnstoff mindestens 1 Mol Amin zur Umsetzung gelangt, dadurch gekennzeichnet, dass man die Umsetzung in Gegenwart von 20 bis 95 Gew.-%, bezogen auf die Gesamtmenge aller Reaktionspartner und Hilfsmittel, einer unter den Reaktionsbedingungen inerten, unter Normaldruck oberhalb der Reaktionstemperatur siedenden organischen Flüssigkeit durchführt, die für den Harnstoff und das Verfahrensprodukt ein Dispergier- oder Lösungsmittel und für das Amin ein Lösungsmittel darstellt,

2. Verfahren gemäss Anspruch 1, dadurch gekennzeichnet, dass man als Amin Dimethyl- oder Diethylamin verwendet.

**Claims**

1. A process for the preparation of alkyl-substituted ureas of the formula:

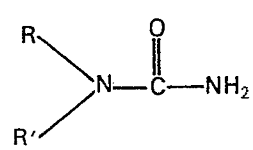

wherein

R and R′, which may be the same or different, each represents or a saturated aliphatic hydrocarbon radical; provided that at least one thereof represents a saturated aliphatic hydrocarbon radical; by the unpressurised reaction, at from 110 to 170 °C, of urea with an amine having a boiling point, under normal pressure, at least 10 °C below the reaction temperature, the amine having the formula:

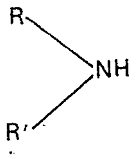

wherein
R and R' as defined above;
the amounts of the reactants being chosen so that, for each mole of urea present, at least one mole of amine is used, characterized in that the reactions carried out in the presence of from 20 to 95%, by weight, based on the total quantity of the reactants and auxiliary agents, of an organic liquid boiling, under normal pressure, above the reaction temperature, which is inert under the reaction conditions and which serves as a dispersing agent or a solvent for the urea and for the product of the process and is a solvent for the amine.

2. A process according to claim 1, characterised in that dimethylamine or diethylamine is used as the amine.

**Revendications**

1. Procédé pour la préparation d'urées alkyl-substituées de formule

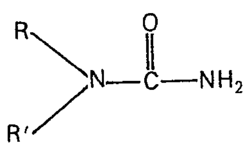

dans laquelle
R et R' représentent des restes identiques ou différents et sont l'hydrogène ou des restes d'hydrocarbures aliphatiques saturés, mais l'un au moins des restes représente un reste d'hydrocarbure aliphatique saturé, par réaction sans pression à 110–170°C de l'urée avec une amine, ayant sous pression normale un point d'ébullition se situant à au moins 10°C au-dessous de la température de réaction, de formule

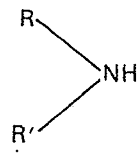

dans laquelle
R et R' ont la signification indiquée ci-dessus, les proportions des partenaires de réaction étant choisies de telle sorte qu'une mole d'amine au moins réagisse pour chaque mole d'urée, caractérisé en ce que l'on effectue la réaction en présence de 20 à 95% en poids, par rapport à la quantité totale de tous les partenaires de réaction et agents auxiliaires, d'un liquide organique inerte dans les conditions de réaction, bouillant sous pression normale au-dessous de la température de réaction, qui soit un agent dispersant ou solvant pour l'urée et le produit du procédé et un solvant pour l'amine.

2. Procédé selon la revendication 1, caractérisé en ce qu'on utilise comme amine la diméthylamine ou la diéthylamine.